# EUROPEAN PATENT SPECIFICATION

(11) **EP 3 057 450 B1**
(45) Date of publication and mention of the grant of the patent: **05.06.2019**
(21) Application number: 14806412.4
(22) Date of filing: 14.10.2014
(51) Int. Cl.: A23L 33/00, A23C 9/142, A23C 9/20

(54) **FOOD COMPOSITION**
LEBENSMITTELZUSAMMENSETZUNG
COMPOSITION ALIMENTAIRE

(30) Priority: 14.10.2013 IT MI20131690
(43) Date of publication of application: 24.08.2016
(73) Proprietor: Consiglio Nazionale Delle Ricerche, 00185 Roma (IT); Università Degli Studi Di Torino, 10124 Torino (IT); Conti, Amedeo, 14022 Castelnuovo Don Bosco (Asti) (IT); Moro, Guido, 20090 Segrate (Milano) (IT)
(72) Inventor: CONTI, Amedeo, I-14022 Castelnuovo Don Bosco (Asti) (IT); MORO, Guido, I-20090 Segrate (Milano) (IT); CAVALLARIN, Laura, I-10125 Torino (IT); BERTINO, Enrico, I-10133 Torino (IT)
(74) Representative: Comoglio, Elena
(86) International application number: PCT/IB2014/065295
(87) International publication number: WO 2015/056166

(56) References cited:
- EP-A1- 2 248 908
- EP-A1- 2 617 290
- CN-B- 102 429 031

## Description

### Field of the invention

The invention relates to a food composition derived from ass's milk, its use in the feeding of weak and/or sensitive subjects, such as premature babies, convalescent subjects and/or the elderly. The invention also relates to a method for manufacturing such a composition and to a method for the administration thereof.

### State of the art

The feeding of weak and/or sensitive subjects, such as premature babies, delicate and convalescent subjects, and/or the elderly, poses several difficulties and must take into account the needs of these categories of living beings.

By way of example, the feeding of premature babies represents a topic of vast research in the fields of medicine and neonatal feeding. The complexity in the feeding of premature babies is connected with several aspects that will affect the baby's health and quality of life in the short and long term. Therefore, qualitatively and quantitatively inappropriate neonatal feeding will result in nutritional deficiencies in the short run and postnatal growth deficiencies, for example neurocognitive impairment, in the long run.

The feeding of premature babies is particularly problematic due to their metabolic condition. In fact, premature babies have low energy supplies, a high metabolic activity, a higher protein turnover, greater glucose and lipid requirements, an inadequacy of the sucking and swallowing reflexes, a diminished gastric capacity, a restricted production of digestive enzymes and growth factors, a decreased bowel peristaltic activity, a high incidence of hypoxaemia and sepsis. A possible and frequent predisposition to develop food allergies must be added to these aspects.

Compositions to be used as a breast milk supplement, conventionally designated as "fortifiers", are known and commercially available. These fortifiers comprise variable amounts of proteins, carbohydrates, calcium, phosphorous, oligominerals, vitamins, fats and electrolytes.

Such fortifiers are often added with protein supplements in variable amounts depending on the baby's needs. Both of the currently commercially available products, the fortifier and the protein supplement, are obtained by processing cow's milk with the addition of synthetic and plant components, even though analogous products derived from human milk are known, but these are too expensive to be put on the market.

Cow's milk has a nutritional composition, and particularly a protein composition, very different from that of human milk, especially from the qualitative point of view. Thus, cow's milk and the components derived therefrom often may be unsuitable for the feeding of premature babies as they may cause sensitization phenomena, which are precursors of allergies and intolerances.

Furthermore, the fortifiers that are presently on the market are formulated as a combination of the required nutrients (proteins, carbohydrates, calcium, phosphate, vitamins and traces of oligoelements) and may be deficient in specific biological activities and in a few essential nutritional elements.

Therefore, there is a need to provide an adequate nutritional supplement yielding a suitable protein intake, which is made of high quality nutritional components and above all is well tolerated by the premature baby.

Delicate subjects, convalescent subjects and the elderly represent a population of living beings which need a specific diet that improves their condition.

EP 2 617 290 A1 discloses an integrated process to produce donkey milk powder products to be used for the nutritional need of infants and elderly people and adults with specific diet requirements.

### Objects of the invention

One object of the present invention is to provide a food composition designed to be used as a breast milk supplement in the feeding of weak and/or sensitive subjects.

Another object of the present invention is to provide a method for manufacturing the said composition.

A further object of the present invention is to provide a composition to be used as a breast milk supplement in the feeding of premature babies, which is obtainable by the method of the invention.

Another object of the present invention is to provide a composition to be used as food or as a food supplement for subjects who need a major energy intake.

A further object of the present invention is to provide a food composition to be used as food or as a food supplement for weak, delicate or convalescent subjects who need a particularly fortifying energy intake.

Another object of the present invention is to provide a food composition to be used as food or as a food supplement for the elderly.

These and still other objects, as well as the related advantages that will be better understood from the following description, are attained by a composition of the invention, which overcomes the limits of the present state of the art and exhibits excellent characteristics of tolerability in premature babies and an ideal nutritional intake.

### Description of the invention

It has now surprisingly been found that it is possible to obtain a mixture of nutrients from ass's milk, which is particularly suitable for the feeding of weak and/or sensitive subjects, such as premature babies, delicate or convalescent subjects, and also the elderly.

In fact, it has been found that a composition based on ass's milk-derived components improves the quality of the baby's growth in terms of the optimization of the clinical, auxological, metabolic and neurological outcome, in the short and long term.

Thus, according to one of its aspects, the present invention relates to a food composition characterized by being derived from ass's milk, which may be used in the feeding of premature babies or in the feeding of particularly sensitive or weak subjects.

The composition according to the invention can also be referred to in the present description as a concentrated multi-component "fortifier", which is a term commonly used for marketed preparations designed for the same purpose.

Here, the expression "premature babies" refers to babies with a body weight of 1500 g or less and/or those with a gestational age of less than 32 weeks.

The term "subjects", according to the present invention, refers to any living being, preferably mammals and even more preferably human beings.

Here, "composition" of the invention intends to denote a mixture of nutritional components derived from the processing of ass's milk.

The composition according to the present invention comprises at least: 20-50 g of proteins, 0.1-25 g of lipids, 3.0-20 g of carbohydrates, 1.4-1.6 g of calcium, and 0.9-1.1 of phosphorous for 100 g of the composition.

According to a particularly preferred embodiment, the composition comprises at least 30-45 g of proteins, 0.5-21 g of lipids, 5-18 g of carbohydrates, 1.5 g of calcium, and 1 g of phosphorous for 100 g of the composition.

According to an even more preferred embodiment, the composition comprises approximately 35 g of proteins, 7 g of carbohydrates, 14 g of lipids, 1.5 g of calcium, and 1 g of phosphorous for 100 g of the concentrated multi-component.

The amount of the components in the composition of the invention, which is indicated in the present description, refers to the percentages of the components for 100 g of the composition in the form of a powder, preferably a lyophilized powder.

The composition may include the addition of a concentrated multi-component, again derived from ass's milk.

The said concentrated multi-component comprises at least 70-90 g of proteins, 0.1-5.0 g of lipids, 2.0-10 g of carbohydrates, 0.0-1.5 g of calcium and 0.0-0.8 g of phosphorous, for 100 g of the concentrated multi-component.

Preferably, the said concentrated multi-component comprises at least 75-88 g of proteins, 0.3-4.8 g of lipids, 2.8-8.7 g of carbohydrates, 0.5-1.2 g of calcium and 0.3-0.65 g of phosphorous, for 100 g of the concentrated multi-component.

According to an even more preferred embodiment, the said concentrated multi-component comprises approximately 80 g of proteins, 6 g of carbohydrates, ≤ 2 g of lipids, 1 g of calcium and 0.4 g of phosphorous, for 100 g of the concentrated multi-component.

The characteristics of the proteins, the lipids and the carbohydrates in the composition and in the concentrated multi-component are described in the following and form part of the invention.

The above-described concentrated multi-component is a further aspect of the present invention and may be added, depending on the needs, to the composition of the invention.

According to a particularly preferred embodiment, the subject-matter of the invention is the composition including the addition of the concentrated multi-component.

The composition, optionally with the addition of the concentrated multi-component, both being derived from ass's milk, overcomes the limits of the present state of the art and gives significant advantages owing to its qualitative composition that is decidedly different from the commercially available fortifiers.

The composition and the concentrated multi-component, subject-matter of the present invention, unlike all the fortifiers based on cow's milk, comprise all the components of the milk from which they derive, simply concentrated by ultrafiltration, thereby retaining all the biological functions typical of whole milk. As said, the marketed products are indeed artificial mixtures of individual ingredients of animal (proteins, which often are hydrolyzed) and plant (fats and carbohydrates) origin, which often require the addition of an emulsifier for their reconstitution and therefore have no enzymatic activity.

Both caseins and whole seroproteins are present in the protein fraction of the ass's milk-derived composition and concentrated multi-component. Particularly, the caseins included comprise: ass's α-s1-casein, ass's α-s2-casein, ass's β-casein, ass's k-casein. The said proteins, the amino acid sequence of which is known, exhibit a higher homology with the corresponding human milk proteins, unlike those of cow's milk contained in the products that are available now.

As to the seroproteic fraction of the composition and concentrated multi-component, it comprises high amounts of ass's lactoferrin, lysozyme and lactoperoxidase, α-lactalbumin and β-lactoglobulin. The said seroproteins are only present in traces in the commercially available fortifiers derived from cow's milk.

The composition and the concentrated multi-component according to the present invention, are different from the marketed products also in the lipid fraction. The lipid fraction is not always present in the currently marketed fortifiers and, when present, it is often of plant origin, such as for instance safflower oil, soybean oil, coconut oil, oils that are poorly tolerated by premature babies.

The ass's milk-derived composition and concentrated multi-component have a high ratio of PUFAs (18% of total fats), 8% of which are n-3 PUFAs, the beneficial nutritional properties of which are known. Approximately 90% of the PUFA content is represented by the n-3 and n-6 series of fatty acid precursors.

The advantages of the composition, either alone or added with the concentrated multi-component, are also found in the carbohydrate fraction. The carbohydrates contained in the currently marketed fortifiers predominantly consist of plant-derived maltodextrins, polysaccharides obtained through enzymatic hydrolysis of corn starch, corn or rice syrup. The said carbohydrates are highly unbearable and poorly digestible and assimilable by premature babies because of the purification processes to which they are subjected. Instead, the composition and the concentrated multi-component of the present invention contain lactose as the main carbohydrate source, which is also the main sugar in human milk.

The composition, optionally with the addition of the concentrated multi-component, according to the invention, besides representing an appropriate source of all the nutritional components required for the feeding of premature babies, including all the essential amino acids, also guarantees an antibacterial action thanks to the high amount of lysozyme.

The composition of the invention, although it has been essentially conceived for the feeding of premature babies, may also be successfully used in the feeding of subjects who need a major intake of components that are fortifying and beneficial for the body, such as weak, delicate or convalescent subjects and/or the elderly, whether they are human beings or animals.

According to another of its aspects, a further subject-matter of the present invention is a method for the manufacturing of the said ass's milk-derived composition and concentrated multi-component.

The method according to the invention comprises the following steps:
a) heating the ass's milk to a temperature between 62°C and 75°C;
b) cooling the ass's milk from step a) to a temperature between 22°C and 45°C;
c) ultrafiltrating the ass's milk from step b) on membranes with a molecular cut off between 5KDa and 50KDa until a product with a protein concentration between 20% and 90% is obtained;
d) heating the product from step c) to a temperature between 62°C and 75°C;
e) allowing the thus obtained composition or concentrated multi-component to cool down and packaging it.

In the method, the ass's milk from step a) is selected from whole ass's milk or skimmed ass's milk, depending on the desired final protein content in the composition.

As regards the product obtained in step c), when it is desired to obtain the composition, the procedure will be continued until a protein concentration between 20 and 50% is obtained, whereas when it is desired to obtain the concentrated multi-component, the procedure will be continued until a protein concentration between 70% and 90% is obtained.

According to a preferred embodiment, in step e) for instance it is possible to cool down the product from step d) at -20°C and freeze-dry it or, alternatively, to cool down the product from step d) to room temperature and package it in a liquid form.

For its use in the feeding of premature babies, the composition, optionally with the addition of the concentrated multi-component, is dissolved or in any case diluted in a liquid, for example in water or milk. Preferably, the said liquid is selected from human milk, ass's milk, formula milk, even more preferably the said liquid is human milk.

The composition can be dissolved and added to a liquid according to the judgement of the neonatologist. By way of example, the said composition can be added to a liquid at a concentration within the range from 0.5% to 5% (weight/volume of the composition in the liquid), preferably in milk, conveniently in human milk.

The concentrated multi-component can be added to the milk, in addition to the composition, according to the individual needs that are defined on the basis of periodic assessments of various metabolic parameters in the newborn baby, such as for example blood urea nitrogen (BUN), preferably in weight/volume ratios ranging from 0.2 to 2%.

However, different doses of the multi-component composition and concentrated multi-component may be envisaged and established by the physician skilled in the art.

A further subject-matter of the invention is a method for the feeding of premature babies, which comprises administering to said newborn babies a certain amount of the composition, optionally with the addition of the concentrated multi-component, dissolved or in any case diluted in a liquid as indicated above.

The present invention is better illustrated by the following examples that in no way take on a limiting value.

### Example 1: Preparation of the composition

Whole ass's milk is treated by heating at a temperature between 62°C and 75°C for a time that ranges from 15 seconds to 15 minutes. It is then allowed to cool down to a temperature between 22°C and 45°C; after reaching such a temperature, the milk is ultrafiltered stepwise through porous membranes with a molecular cut off between 5KDa and 50KDa.

The ultrafiltration operating conditions comprise an inward pressure ranging from 1 to 4 bars, an outward pressure ranging from 1 to 3,5 bars, with a permeate flow between 50 and 200 L/h.

At the end of the ultrafiltration process, once a protein concentration in the retentate of between 20 and 50% is reached, the retentate is treated once more by heating at a temperature between 62°C and 75°C for a time that ranges from 15 seconds to 15 minutes and then immediately cooled down to -20°C, to then be vacuum freeze-dried or, alternatively, packaged as a liquid for example and preferably in a sterile environment.

### Example 2: Preparation of the concentrated multi-component

Skimmed ass's milk is treated by heating at a temperature between 62°C and 75°C for a time that ranges from 15 seconds to 15 minutes. It is then allowed to cool down to a temperature between 22°C and 45°C; after reaching such a temperature, the milk is ultrafiltered stepwise through porous membranes with a molecular cut off between 5Da and 50KDa.

The ultrafiltration operating conditions comprise an inward pressure ranging from 1 to 4 bars, an outward pressure ranging from 1 to 3.5 bars, with a permeate flow between 50 and 200 L/h.

At the end of the ultrafiltration process, once a protein concentration in the retentate of between 70 and 90% is reached, the retentate is treated once more by heating at a temperature between 62°C and 75°C for a time that ranges from 15 seconds to 15 minutes and then immediately cooled down to -20°C, to then be vacuum freeze-dried.

### Example 3: Characterization of the protein profiles of the ass's milk-based composition and concentrated multi-component and of the marketed products based on cow's milk

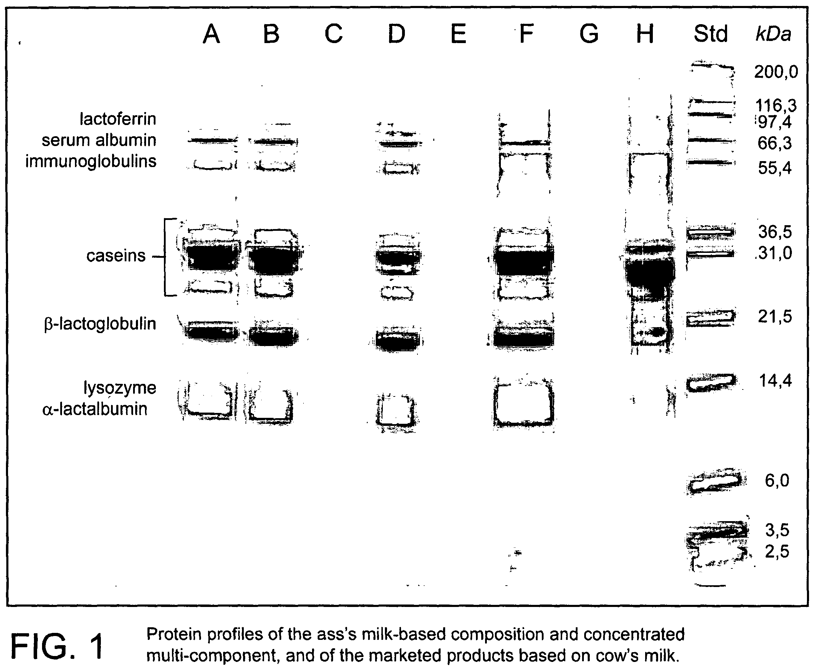

The protein profiles of ass's milk, and ass's milk-based concentrated multi-component and concentrate, and of the corresponding commercial products based on cow's milk, used in the clinical trial, are shown in Figure 1. In the product of the present invention (A, B, D) there are whole proteins with a profile entirely similar to that of the original ass's milk (F). The concentrated multi-component based on cow's milk (G) is totally lacking whole proteins, as they were extensively hydrolyzed during the manufacturing process, and as such, does not have the beneficial biological activities associated therewith. In the cow's milk-based concentrate (H) there are predominantly caseins and bovine β-lactoglobulin, which are well-known allergenic proteins, whereas in contrast there is a feeble presence of the "noble" proteins alpha lactalbumin and lysozyme, much more evident in the concentrated multi-component, and concentrate, based on ass's milk.

The letters in Figure 1 are related to the following key:

### Key

A: Ass's milk concentrated multi-component (10 µg proteins).
B: 2x Ass's milk concentrated multi-component (10 µg proteins).
C: Sample B permeate.
D: 4x Ass's milk concentrated multi-component (10 µg proteins).
E: Sample D permeate.
F: Ass's milk only (10 µg proteins).
G: Concentrated multi-component FM85 (Nestle) (10 µg proteins).
H: Protein concentrate Protifar (Nutricia) (10 µg proteins).
STD: molecular weight standard Invitrogen.

## Claims

1. An ass's milk-derived food composition, **characterized in that** it comprises at least: 20-50 g of proteins, 0.1-25 g of lipids, 3.0-20 g of carbohydrates, 1.4-1.6 g of calcium, and 0.9-1.1 of phosphorous for 100 g of the composition.

2. The composition according to claim 1, **characterized in that** it comprises at least 30-45 g of proteins, 0.5-21 g of lipids, 5-18 g of carbohydrates, 1.5 g of calcium, and 1 g of phosphorous for 100 g of the composition.

3. The composition according to any of claims 1 to 2, **characterized in that** it comprises 35 g of proteins, 7 g of carbohydrates, 14 g of lipids, 1.5 g of calcium, and 1 g of phosphorous for 100 g of the composition.

4. The composition according to any claims 1 to 3, **characterized in that** it is with the addition of a concentrated multi-component derived from ass's milk.

5. An ass's milk-derived concentrated multi-component, which comprises at least 70-90 g of proteins, 0.1-5.0 g of lipids, 2.0-10 g of carbohydrates, 0.0-1.5 g of calcium, 0.0-0.8 g of phosphorous, for 100 g of the concentrated multi-component.

6. The concentrated multi-component according to claim 5, **characterized in that** it comprises at least 75-88 g of proteins, 0.3-4.8 g of lipids, 2.8-8.7 g of carbohydrates, 0.5-1.2 g of calcium and 0.3-0.65 g of phosphorous, for 100 g of the concentrated multi-component.

7. The concentrated multi-component according to any of claims 5 or 6, **characterized in that** it comprises approximately 80 g of proteins, 6 g of carbohydrates, ≤ 2 g of lipids, 1 g of calcium and 0.4 g of phosphorous, for 100 g of the concentrated multi-component.

8. A method for the manufacturing of the composition according to any of claims 1-5 or of the concentrated multi-component according to any of claims 5-7, **characterized in that** it comprises the following steps:
a) heating the ass's milk to a temperature between 62°C and 75°C;
b) cooling the ass's milk from step a) to a temperature between 22°C and 45°C;
c) ultrafiltrating the ass's milk from step b) on membranes with a molecular cut off between 5KDa and 50KDa until a product with a protein concentration between 20% and 90% is obtained;
d) heating the product from step c) to a temperature between 62°C and 75°C;
e) allowing the thus obtained composition or concentrated multi-component to cool down and packaging it.

9. The method according to claim 8, wherein the said ass's milk from step a) is selected from whole ass's milk and skimmed ass's milk.

10. The method according to claims 8 or 9, wherein the said product from step c) has a protein concentration ranging from 20% to 50%.

11. The method according to claims 8 or 9, wherein the said product from step c) has a protein concentration ranging from 70% to 90%.

12. The use of the composition according to any of claims 1-4, optionally with the addition of the concentrated multi-component according to any of claims 5-7, for the feeding of premature babies.

13. The use of the composition according to any of claims 1-4, optionally with the addition of the concentrated multi-component according to any of claims 5-7, for the feeding of weak and/or delicate and/or convalescent subjects and/or of the elderly.

14. A method for the feeding of premature babies, **characterized in that** it comprises administering to a subject a composition according to any of claims 1-4, optionally with the addition of the concentrated multi-component according to any of claims 5-7, dissolved and/or diluted in a liquid.

15. The method according to claim 14, wherein the said liquid is selected from human milk, ass's milk or formula milk.

## Patentansprüche

1. Lebensmittelzusammensetzung, die aus Eselsmilch gewonnen ist, **dadurch gekennzeichnet, dass** sie zumindest umfasst: 20-50 g an Proteinen, 0,1-25 g an Lipiden, 3,0-20 g an Kohlenhydraten, 1,4-1,6 g an Calcium und 0,9-1,1 g an Phosphor bezogen auf 100 g der Zusammensetzung.

2. Zusammensetzung nach Anspruch 1, **dadurch gekennzeichnet, dass** sie zumindest 30-45 g an Proteinen, 0,5-21 g an Lipiden, 5-18 g an Kohlenhydraten, 1,5 g an Calcium und 1 g an Phosphor bezogen auf 100 g der Zusammensetzung umfasst.

3. Zusammensetzung nach einem der Ansprüche 1 bis 2, **dadurch gekennzeichnet, dass** sie 35 g an Proteinen, 14 g an Lipiden, 7 g an Kohlenhydraten, 1,5 g an Calcium und 1 g an Phosphor bezogen auf 100 g der Zusammensetzung umfasst.

4. Zusammensetzung nach einem der Ansprüche 1 bis 3, **dadurch gekennzeichnet, dass** sie mit dem Zusatz einer konzentrierten Mehrfachkomponentenbeigabe versehen ist, die aus Eselsmilch gewonnen ist.

5. Konzentrierte Mehrfachkomponentenbeigabe, die aus Eselsmilch gewonnen ist, welche zumindest 70-90 g an Proteinen, 0,1-5,0 g an Lipiden, 2,0-10 g an Kohlenhydraten, 0,0-1,5 g an Calcium, 0,0-0,8 g an Phosphor bezogen auf 100 g der konzentrierten Mehrfachkomponentenbeigabe umfasst.

6. Konzentrierte Mehrfachkomponentenbeigabe nach Anspruch 5, **dadurch gekennzeichnet, dass** sie zumindest 75-88 g an Proteinen, 0,3-4,8 g an Lipiden, 2,8-8,7 g an Kohlenhydraten, 0,5-1,2 g an Calcium und 0,3-0,65 g an Phosphor bezogen auf 100 g der konzentrierten Mehrfachkomponentenbeigabe umfasst.

7. Konzentrierte Mehrfachkomponentenbeigabe nach einem der Ansprüche 5 oder 6, **dadurch gekennzeichnet, dass** sie etwa 80 g an Proteinen, ≤ 2 g an Lipiden, 6 g an Kohlenhydraten, 1 g an Calcium und 0,4 g an Phosphor bezogen auf 100 g der konzentrierten Mehrfachkomponentenbeigabe umfasst.

8. Verfahren zur Herstellung der Zusammensetzung nach einem der Ansprüche 1-5 oder der konzentrierten Mehrfachkomponentenbeigabe nach einem der Ansprüche 5-7, **dadurch gekennzeichnet, dass** es die folgenden Schritte umfasst:
a) Erhitzen der Eselsmilch auf eine Temperatur zwischen 62 °C und 75 °C;
b) Abkühlen der Eselsmilch von Schritt a) auf eine Temperatur zwischen 22 °C und 45 °C;
c) Ultrafiltration der Eselsmilch von Schritt b) auf Membranen mit einem molekularen Gewichtsgrenzwert zwischen 5 KDa und 50 KDa bis ein Produkt mit einer Proteinkonzentration zwischen 20 % und 90 % erhalten wird;
d) Erhitzen des Produktes von Schritt c) auf eine Temperatur zwischen 62 °C und 75 °C;
e) Erlauben der somit erhaltenen Zusammensetzung oder konzentrierten Mehrfachkomponentenbeigabe abzukühlen und Verpacken derselben.

9. Verfahren nach Anspruch 8, wobei die Eselsmilch von Schritt a) ausgewählt ist aus Eselsvollmilch und entrahmter Eselsmilch.

10. Verfahren nach Anspruch 8 oder 9, wobei das Produkt von Schritt c) eine Proteinkonzentration von 20 % bis 50 % aufweist.

11. Verfahren nach Anspruch 8 oder 9, wobei das Produkt von Schritt c) eine Proteinkonzentration von 70 % bis 90 % aufweist.

12. Verwendung der Zusammensetzung nach einem der Ansprüche 1-4, wahlweise mit der Zugabe der konzentrierten Mehrfachkomponentenbeigabe nach einem der Ansprüche 5-7, für die Ernährung von Frühgeburten.

13. Verwendung der Zusammensetzung nach einem der Ansprüche 1-4, wahlweise mit der Zugabe der konzentrierten Mehrfachkomponentenbeigabe nach einem der Ansprüche 5-7, für die Ernährung von schwachen und/oder empfindlichen und/oder genesenden Subjekten und/oder von Älteren.

14. Verfahren für die Ernährung von Frühgeburten, **dadurch gekennzeichnet, dass** es das Verabreichen einer Zusammensetzung nach einem der Ansprüche 1-4 an ein Subjekt umfasst, wahlweise mit der Zugabe der konzentrierten Mehrfachkomponentenbeigabe nach einem der Ansprüche 5-7, aufgelöst und/oder verdünnt in einer Flüssigkeit.

15. Verfahren nach Anspruch 14, wobei die Flüssigkeit ausgewählt ist aus Menschenmilch, Eselsmilch oder Folgemilch.

## Revendications

1. Composition alimentaire dérivée de lait d'ânesse, **caractérisée en ce qu'**elle comprend au moins : 20 à 50 g de protéines, 0,1 à 25 g de lipides, 3,0 à 20 g de glucides, 1,4 à 1,6 g de calcium et 0,9 à 1,1 g de phosphore pour 100 g de la composition.

2. Composition selon la revendication 1, **caractérisée en ce qu'**elle comprend au moins 30 à 45 g de protéines, 0,5 à 21 g de lipides, 5 à 18 g de glucides, 1,5 g de calcium et 1 g de phosphore pour 100 g de la composition.

3. Composition selon l'une quelconque des revendications 1 à 2, **caractérisée en ce qu'**elle comprend 35 g de protéines, 7 g de glucides, 14 g de lipides, 1,5 g de calcium et 1g de phosphore pour 100 g de la composition.

4. Composition selon l'une quelconque des revendications 1 à 3, **caractérisée en ce qu'**elle est avec l'ajout d'un concentré multi-composant dérivé de lait d'ânesse.

5. Concentré multi-composant dérivé de lait d'ânesse, qui comprend au moins 70 à 90 g de protéines, 0,1 à 5,0 g de lipides, 2,0 à 10 g de glucides, 0,0 à 1,5 g de calcium, 0,0 à 0,8 g de phosphore, pour 100 g du concentré multi-composant.

6. Concentré multi-composant selon la revendication 5, **caractérisé en ce qu'**il comprend au moins 75 à 88 g de protéines, 0,3 à 4,8 g de lipides, 2,8 à 8,7 g de glucides, 0,5 à 1,2 g de calcium et 0,3 à 0,65 g de phosphore, pour 100 g du concentré multi-composant.

7. Concentré multi-composant selon l'une quelconque des revendications 5 ou 6, **caractérisé en ce qu'**il comprend approximativement 80 g de protéines, 6 g de glucides, ≤ 2 g de lipides, 1 g de calcium et 0,4 g de phosphore, pour 100 g du concentré multi-composant.

8. Procédé pour la fabrication de la composition selon l'une quelconque des revendications 1 à 5 ou du concentré multi-composant selon l'une quelconque des revendications 5 à 7, **caractérisé en ce qu'**il comprend les étapes suivantes :
a) chauffer le lait d'ânesse à une température entre 62 °C et 75 °C ;
b) refroidir le lait d'ânesse issu de l'étape a) à une température entre 22°C et 45 °C ;
c) soumettre à une ultrafiltration le lait d'ânesse issu de l'étape b) sur des membranes ayant un seuil de coupure moléculaire entre 5 KDa et 50 KDa jusqu'à ce qu'un produit ayant une concentration en protéines entre 20 % et 90 % soit obtenu ;
d) chauffer le produit issu de l'étape c) à une température entre 62 °C et 75 °C ;
e) laisser la composition ou le concentré multi-composant ainsi obtenu refroidir et l'emballer.

9. Procédé selon la revendication 8, dans lequel ledit lait d'ânesse de l'étape a) est sélectionné parmi le lait d'ânesse entier et le lait d'ânesse écrémé.

10. Procédé selon les revendications 8 ou 9, dans lequel ledit produit issu de l'étape c) a une concentration en protéines allant de 20 % à 50%.

11. Procédé selon les revendications 8 ou 9, dans lequel ledit produit issu de l'étape c) a une concentration en protéines allant de 70 % à 90%.

12. Utilisation de la composition selon l'une quelconque des revendications 1 à 4, facultativement avec l'ajout du concentré multi-composant selon l'une quelconque des revendications 5 à 7, pour l'alimentation de bébés prématurés.

13. Utilisation de la composition selon l'une quelconque des revendications 1 à 4, facultativement avec l'ajout du concentré multi-composant selon l'une quelconque des revendications 5 à 7, pour l'alimentation des sujets affaiblis et/ou fragiles et/ou convalescents et/ou des personnes âgées.

14. Procédé pour l'alimentation de bébés prématurés, **caractérisé en ce qu'**il comprend l'administration à un sujet d'une composition selon l'une quelconque des revendications 1 à 4, facultativement avec l'ajout du concentré multi-composant selon l'une quelconque des revendications 5 à 7, dissoute et/ou diluée dans un liquide.

15. Procédé selon la revendication 14, dans lequel ledit liquide est sélectionné parmi le lait humain, le lait d'ânesse ou le lait artificiel.
